# EUROPÄISCHE PATENTSCHRIFT

(11) **EP 1 924 296 B1**
(45) Veröffentlichungstag und Bekanntmachung des Hinweises auf die Patenterteilung: **18.02.2009**
(21) Anmeldenummer: 06778096.5
(22) Anmeldetag: 01.08.2006
(51) Int. Cl.: A61L 15/40, A61K 36/75, A61K 36/22, A61K 36/28

(54) **VERBANDSMATERIAL MIT WIRKSTOFFEN**
DRESSING MATERIAL COMPRISING ACTIVE SUBSTANCES
MATERIAU DE BANDAGE A PRINCIPES ACTIFS

(30) Priorität: 13.09.2005 EP 05019854
(43) Veröffentlichungstag der Anmeldung: 28.05.2008
(73) Patentinhaber: Hohmann, Sabine, 69121 Heidelberg (DE)
(72) Erfinder: Hohmann, Sabine, 69121 Heidelberg (DE)
(74) Vertreter: Dick, Alexander
(86) Internationale Anmeldenummer: PCT/EP2006/064887
(87) Internationale Veröffentlichungsnummer: WO 2007/031364

(56) Entgegenhaltungen:
- US-A- 5 843 523
- PUNZO A: "Homeopathic medicine: An overview with specific applications for the orthopaedic patient" TECHNIQUES IN ORTHOPAEDICS 2003 UNITED STATES, Bd. 18, Nr. 1, 2003, Seiten 54-61, XP009078704 ISSN: 0885-9698
- DATABASE INTERNET [Online] XP002419378 gefunden im HTTP://WWW.WRC.NET/WRCNET_CONTENT/ENCYCLOP EDIA.ASPX?AID=135

## Beschreibung

Die vorliegende Erfindung betrifft ein Verbandsmaterial umfassend ein Trägermaterial sowie zumindest einen Wirkstoff aus jeweils Arnica montana, Rhus toxicodendron und Ruta graveolens. Ferner betrifft die Erfindung ein Arzneimittel umfassend dieses Verbandsmaterial sowie die Verwendung dieses Verbandsmaterials zur Herstellung eines Arzneimittels zur Behandlung von Erkrankungen oder Störungen des Bewegungsapparates. Vorzugsweise wird das Verbandsmaterial als Tapeverband hierbei eingesetzt.

Verletzungen des Bewegungsapparates, z.B. Kapselbandverletzungen, Muskelsehnenverletzungen, knöcherne Verletzungen oder auch degenerative Prozesse, können durch teilweise oder vollständige Ruhigstellung der betroffenen Körperpartien behandelt werden. Die vollständige Ruhestellung, z.B. durch Gipsverbände, ist im Stand der Technik wohlbekannt. Ein Nachteil dieser Behandlungsmethode ist jedoch, dass die betroffenen Körperpartien oft über einen längeren Zeitraum ruhig gestellt werden müssen und durch die Art der Ruhigstellung, z.B. Gipsverband, Folgeschäden an den Körperpartien auftreten können, wie Durchblutungsstörungen, verminderte Lymphdrainage oder auch Gewebeatrophien. Neben der vollständigen Ruhigstellung von betroffenen Körperpartien gibt es mittlerweile auch Verfahren zur teilweisen Ruhigstellung. Von besonderer Bedeutung sind hierbei die Tapeverbände, durch die verschiedene Verletzungen in kurzer Zeit zur Ausheilung gebracht werden können, ohne dass es einer vollständigen Ruhigstellung (z.B. durch Gips) bedarf. Zumindest einige der Folgeschäden, die durch die bekannten Maßnahmen zur vollständigen Ruhigstellung von Körperpartien auftreten können, können dadurch effizient vermieden werden. Der Tapeverband stützt und entlastet geschädigte Anteile, z.B. eines Gelenks, für Bewegungen, erlaubt eine funktionelle Belastung in begrenztem Ausmaß und vermeidet dadurch extreme Bewegungen, die unter Umständen zu einer weiteren Schädigung verschiedener Gelenkanteile führen könnten. Er ermöglicht jedoch funktionelle Nachbehandlungen, z.B. nach einer Gelenkoperation. Tapeverbände haben dabei nicht nur stabilisierende Wirkung als passive äußere Hülle, sondern aktivieren auch Muskelgruppen über die Reizung von Mechanorezeptoren in der Haut sowie Propriorezeptoren von Muskeln und Sehnen. Durch die Anspannung der aktivierten Muskelgruppen wird eine zusätzliche Stabilisierung, auch als propriozeptiver Effekt bezeichnet, bewirkt. Tapeverbände sind also mittlerweile aus der medizinischen Versorgung nicht mehr wegzudenken. Trotzdem können einige der zuvor im Zusammenhang mit Verbänden, die eine vollständige Ruhigstellung von Körperpartie bewirken, genannten Folgeschäden auch bei Tapeverbänden auftreten. Hierzu gehören insbesondere Schäden durch unzureichende Lymphdrainage, Blutversorgung oder Gewebeatrophien.

Aufgabe der vorliegenden Erfindung ist es also, Maßnahmen bereitzustellen, durch die die zuvor genannten Nachteile vermieden werden können, wobei gleichzeitig ein verbesserter Heilungsprozess für geschädigte Körperpartien des Bewegungsapparates ermöglicht wird.

Die Aufgabe wird durch die in den Ansprüchen sowie anschließend beschriebenen Ausführungsformen gelöst.

Die Erfindung betrifft daher ein Verbandsmaterial umfassend ein Trägermaterial sowie zumindest einen Wirkstoff aus jeweils Arnica montana, Rhus toxicodendron und Ruta graveolens. Die vorliegende Erfindung betrifft weiterhin ein Verbandsmaterial umfassend einer Trägermaterial sowie zumindest einen Wirkstoff aus jeweils Flos cathaimi, Ruta graveolens und Rhus delavayi.

Darüber hinaus betrifft die vorliegende Erfindung auch Verbandsmaterialien, die ein Trägermaterial umfassen; vorzugsweise wie zuvor beschrieben, und zumindest einen weiteren Wirkstoff aufweisen, der ausgewählt ist aus der Gruppe bestehend aus: (i) Wirkstoffen aus Bryonia alba, Bryonia dioica, Mixturen aus Kirschpflaumenblüten, Blüten der Weißen Waldrebe, Blüten des Drüsen tragenden Springkrauts, Blüten des gelben Sonnenröschens und Blüten des Goldigen Milchsterns (kommerziell erhältlich als Rescue Remedy Mischung), Hypericum perforatum (Johanniskraut), Apis mellifica, Calendula, Agaricus muscarius, Anacardium orientale, Lendium palustris, Bellis perennsis, Hamamelis viriginica sowie (ii) den Wirkstoffen Strontium carbonicum, Acidum sulforicum und Diclofenac. Bei Diclofenac handelt es sich um ein bekanntes Arzneimittel mit der chemischen Bezeichnung [2-(2,6-Dichloranilino)phenyl]essigsäure-natriumsalz.

Verbandsmaterial im Sinne der vorliegenden Erfindung ist ein flexibles Material, das auf der Haut des Körpers an geschädigten Körperpartien des Bewegungsapparates angebracht werden kann. Die Flexibilität des Verbandsmaterials soll so gewählt sein, dass es einerseits eine geführte, unterstützte Bewegung der betroffenen Teile des Bewegungsapparates gestattet, diesen andererseits aber auch hinreichend fixiert, um unerwünschte oder extreme Bewegungen, wie Überdehnungen, zu vermeiden. Das erfindungsgemäße Verbandsmaterial kann aus einem oder mehreren Materialien aufgebaut sein. Das Verbandsmaterial umfasst zumindest ein Trägermaterial sowie einen Wirkstoff oder eine Wirkstoffkombination wie zuvor beschrieben. Der Wirkstoff kann hierbei direkt auf dem Trägermaterial aufgebracht sein oder an einen Hilfsstoff gekoppelt sein, der auf dem Trägermaterial aufgebracht ist. Neben dem Trägermaterial und den Wirkstoffen kann das Verbandsmaterial somit auch Hilfsstoffe umfassen. Geeignete Hilfsstoffe umfassen pharmazeutisch verträgliche Lösungsmittel für die Wirkstoffe, Stoffe, die die Elastizität oder Steifigkeit des Trägermaterials beeinflussen, aber auch Stoffe, die für die Anbringung des Verbandsmaterials an der Haut benötigt werden, wie z.B. Klebemittel. Das Verbandsmaterial kann in jeder geometrischen Form vorliegen. Bevorzugt liegt es in Form eines rechteckigen oder runden Pflasters oder, besonders bevorzugt, als rechteckiges Band vor. Das erfindungsgemäße Verbandsmaterial ist vorzugsweise luftdurchlässig, wasserabweisend und besteht aus hypoallergenen Materialien. Durch die Auswahl geeigneter Farben, vorzugsweise rot, blau, gelb, kann zusätzlich auf energetischem Weg auf das Körpergeschehen und den Heilungsprozess Einfluss genommen werden. Als Verbandsmaterialien können erfindungsgemäß auch alle kommerziell erhältlichen Pflaster, Verbände oder Tapeverbände eingesetzt werden.

Unter dem Begriff "Trägermaterial" ist im Rahmen der vorliegenden Erfindung ein gewebter oder nicht gewebter Stoff zu verstehen, der aus einem oder mehreren unterschiedlichen Bestandteilen aufgebaut sein kann. Das Grundgerüst des Trägermaterials ist vorzugsweise aus einem natürlichen oder synthetischen Polymer, z.B. einem Polyester, oder einem Polykondensat, z.B. einem Polyhydroxyalkanoat, aufgebaut. Geeignete Stoffe für das Grundgerüst des Trägermaterials sind im Stand der Technik bekannt. Besonders bevorzugt umfasst das Trägermaterial eine Mischung aus natürlichen Bestandteilen, vorzugsweise Baumwolle oder Kautschuk, und synthetischen Bestandteilen, vorzugsweise Acryl. In das Trägermaterial können weitere Stoffe, z.B. die zuvor genannten Hilfsstoffe, eingebracht werden. Das Trägermaterial soll weiterhin so beschaffen sein, dass es entweder direkt oder über Hilfsstoffe eine Verbindung mit den zuvor genannten Wirkstoffen oder Wirkstoffkombinationen eingehen kann. Ebenso soll vorzugsweise das Trägermaterial in der Lage sein, eine Verbindung mit einem Klebemittel einzugehen, wie andernorts in der Beschreibung offenbart.

Der Begriff "zumindest einen Wirkstoff" bezieht sich sowohl auf isolierte Wirkstoffe als auch auf Wirkstoffgemische. Ein Wirkstoff, wie hierin verwendet, ist eine chemische Verbindung, die aus einer der zuvor genannten Pflanzen oder einem Pflanzenteil, z.B. Samen, Blätter oder Stängel, davon gewonnen werden kann. Ein Wirkstoffgemisch im Sinne der vorliegenden Erfindung ist ein Gemisch von solchen chemischen Verbindungen. Das Gemisch kann hierbei durch Vermischung entsprechender Einzelwirkstoffe hergestellt werden oder durch gemeinsame Gewinnung verschiedener Wirkstoffe aus den Pflanzen oder Pflanzenanteilen bereitgestellt werden. Vorzugsweise wird im Rahmen der vorliegenden Erfindung ein Wirkstoffgemisch aus jeweils Arnica montana, Rhus toxicodendron und Ruta graveolens durch Extraktion mit einem Lösungsmittel bestehend aus einem Alkohol und Wasser gewonnen. Hierzu werden zunächst jeweils gesunde Pflanzen geerntet, gesäubert und anschließend frisch zerkleinert. Der zerkleinerte Pflanzenbrei wird nun mit den Lösungsmittel vermischt und vorzugsweise bei Dunkelheit inkubiert, so dass die Wirkstoffe aus den zerkleinerten Pflanzenfragmenten durch das Lösungsmittel herausgelöst werden können. Durch anschließende Trennung der zerkleinerten Pflanzenfragmente von dem Lösungsmittelwirkstoffgemisch wird nun ein Urextrakt gewonnen. Die Trennung kann beispielsweise durch Filtration, Sedimentieren oder Zentrifugation erfolgen. Vorzugsweise wird das Urextrakt durch Filtration gewonnen. Das verwendete Lösungsmittel besteht vorzugsweise zu 90 % aus einem Alkohol und 10 % aus Wasser. Besonders bevorzugt ist der Alkohol Ethanol. Einzelheiten zur Wirkstoffformulierung und Herstellung der Wirkstoffe, die im Rahmen der vorliegenden Erfindung eingesetzt werden sollen, sind dem "Deutschen Homöopathischen Arzneibuch", Schwabe, Leipzig, 1901, (HAB) zu entnehmen. Präparate, die entsprechend hergestellte Wirkstoffe und/oder Wirkstoffgemische enthalten, sind kommerziell erhältlich. Extrakte aus Arnica montana werden vorzugsweise aus den getrockneten unterirdischen Anteilen der Pflanze oder den oberirdischen Anteilen zur Blütezeit nach den Regeln 4a und 17 des HAB gewonnen. Extrakte aus Rhus toxicodendron werden vorzugsweise aus jungen, noch nicht verholzten Sprossen nach Regeln 2a und 7 des HAB gewonnen. Extrakte aus Ruta graveolens werden vorzugsweise aus den frischen, oberirdischen Teilen zur Blütezeit nach den Regeln 3a und 7 des HAB gewonnen. Der mit den zuvor beschriebenen Verfahren hergestellte Urextrakt wird im erfindungsgemäßen Verbandsmaterial vorzugsweise als verdünnter Extrakt eingesetzt. Als Verdünnungsmittel wird hierbei ebenfalls ein Alkoholwassergemisch, vorzugsweise wie oben beschrieben, eingesetzt. Der verdünnte Extrakt wird vorzugsweise durch Verschüttelung, wie im HAB beschrieben, hergestellt.

Alternativ können Wirkstoffe, die nicht in Alkohol, destilliertem Wasser aus dem Rohstoff (z.B. Pflanzenblätter) aufgelöst werden können, zunächst bis zur 3./4. Potenz verrieben (trituriert) werden. Bei der Verreibung wird der Rohstoff je Potenzierungsschritt 3 x mit Milchzucker in einem Mörser verrieben und aufgescharrt, wobei das Verhältnis zwischen Rohstoff und Milchzucker etwa 1:100 betragen sollte. Die zuvor beschriebenen Extrakte oder Triturierungen werden vorzugsweise zur lokalen Anwendung formuliert.

Die Wirkstoffe aus den Pflanzen oder Pflanzenbestandteilen von Fos cathaimi, Rhus Delavayi, Bryonia alba, Bryonia dioica, Johanniskraut, Apis melifica, Calendula, Agaricus muscarius, Anacardium Orientale, Lendium palustris, Bellis perennsis und Hamamelis virginica können ebenfalls entsprechend den Vorschriften HAB gewonnen werden. Besonders bevorzugt können Wirkstoffe aus Bryonia alba oder dioica gemäß Regel 2a des HAB gewonnen werden. Wirkstoffe aus Agaricus muscarius können vorzugsweise nach Regel 3a des HAB gewonnen werden. Wirkstoffe aus Apis melifica können vorzugsweise nach Regel 4b des HAB gewonnen werden.

Besonders bevorzugt werden die Wirkstoffe und Wirkstoffgemische als Salben oder Cremes formuliert. Neben den zuvor genannten Extrakten oder Triturierungen enthalten die Salben oder Cremes vorzugsweise noch Wollwachs, Wollwachsalkohol Salbe (bestehend aus Cetylstearylalkohol, Wollwachsalkoholen und weißem Visilin), dickflüssiges Paraffin und Wasser. Üblicherweise enthalten die Salben den Extrakt oder die Triturierung mit den Wirkstoffen in einer Konzentration von 5-20 % (w/w), vorzugsweise 10 % (w/w). Anstelle von Salbenform können die zuvor genannten Extrakte oder Triturierungen auch in jeder anderen für die lokale Verabreichung geeigneten Form am Trägermaterial angebracht sein, z.B. als Gel. Voraussetzung ist jedoch, dass diese Verabreichungsform eine Bioverfügbarkeit der Wirkstoffe bei lokaler, dermaler Verabreichung gestattet.

Im Rahmen der vorliegenden Erfindung wurde festgestellt, dass ein Verbandsmaterial, welches die zuvor genannten Bestandteile umfasst, die therapeutischen Vorteile eines im Stand der Technik bekannten Tapeverbands besitzt, gleichzeitig aber dessen Nachteile umgehen kann. Durch das zusätzlich Aufbringen von Wirkstoffen z.B. aus Arnica montana, Rhus toxicodendron und Ruta graveolens und die damit ermöglichte lokale Versorgung des mit dem Verbandsmaterial versehenen Abschnitts des Bewegungsapparates wird eine effiziente Lymphdrainage, gesteigerte Blutversorgung (insbesondere venöse Aktivierung), eine Stabilisierung der geschädigten Struktur unter Beibehaltung guter Beweglichkeit, ein Vermeiden von Muskelatrophie sowie eine die Therapie fördernde Beeinflussung von Haut- und Schmerzrezeptoren, Muskel und Muskelansätzen sowie des Kapsel- und des Bandapparates bewirkt. Verletzungen und Störungen von Teilen des Bewegungsapparates wie muskuläre Schmerzsyndrome, Muskelverkrampfungen, Gelenkschmerzen und Gelenkverstauchungen, Sehnen- und Bänderbeschwerden, rheumatische Beschwerden, Prellungen, Überdehnungen, Muskelfaserrisse, Bandrupturen, Zerrungen, frische Verletzungen mit ausgeprägten Hämatomen, Fingerstauchungen, Skidaumen, Tennis- und Golfellenbogen, Inpigmentsyndrome, Insertionsendopathien, Abduktorenverletzungen, Kniegelenksergüsse, Kniegelenksbeschwerden, Achillessehnenbeschwerden, Fersen-sporne, Sprunggelenksbeschwerden, Vor- und Mittelfußschmerzen (Längs- und Quergewölbe), Handgelenksbeschwerden, Halswirbelsäulen-, Brustwirbelsäulen- oder Längswirbelsäulenbeschwerden, Migräne oder Lymphödeme können dank der vorliegenden Erfindung effizient und ohne Nebenwirkungen oder Folgeschäden behandelt werden. In den der vorliegenden Erfindung zugrunde liegenden Fallstudien wurden festgestellt, dass sich die vorteilhaften Effekte eines Verbandsmaterials, z.B. eines Tapeverbands, synergistisch mit den therapeutischen Effekten der zuvor genannten Wirkstoffe kombinieren lassen, wobei zusätzlich zu dem gefundenen Synergismus auch gleichzeitig ein drastischer Rückgang der Folgeschäden, die durch einen Tapeverband ausgelöst werden können, beobachtet wurde.

In einer bevorzugten Ausführungsform des erfindungsgemäßen Verbandsmaterials ist der zumindest eine Wirkstoff aus Arnica montana ein verdünnter Extrakt, der aus einem Teilextrakt auf 10¹² Teilen eines pharmazeutisch verträglichen Lösungsmittels, vorzugsweise eines Alkoholwassergemischs, wie zuvor beschrieben, besteht. Ein solcher verdünnter Extrakt wird im Stand der Technik auch als Arnica D12 bezeichnet und ist kommerziell erhältlich.

In einer bevorzugten Ausführungsform des erfindungsgemäßen Verbandsmaterials ist der zumindest eine Wirkstoff aus Rhus toxicodendron ein verdünnter Extrakt, der aus einem Teilextrakt auf 10¹² Teilen eines pharmazeutisch verträglichen Lösungsmittels, vorzugsweise eines Ethanolwassergemischs, wie zuvor beschrieben, besteht. Ein solcher verdünnter Extrakt wird im Stand der Technik auch als Rhus toxicodendron D12 bezeichnet und ist kommerziell erhältlich.

In einer bevorzugten Ausführungsform des erfindungsgemäßen Verbandsmaterials ist der zumindest eine Wirkstoff aus Ruta graveolens ein verdünnter Extrakt, der aus einem Teilextrakt auf 10¹² Teilen eines pharmazeutisch verträglichen Lösungsmittels, vorzugsweise eines Ethanolwassergemischs, wie zuvor beschrieben, besteht. Ein solcher verdünnter Extrakt wird im Stand der Technik auch als Ruta graveolens D12 bezeichnet und ist kommerziell erhältlich.

In einer weiteren bevorzugten Ausführungsform des erfindungsgemäßen Verbandsmaterials weist dieses ein Klebemittel zum Befestigen des Verbandsmaterials an der Haut auf. Als "Klebemittel" im Sinne der vorliegenden Erfindung können Mittel verwendet werden, die eine reversible Verbindung zwischen der Haut und dem Verbandsmaterial vermitteln. Bevorzugt handelt es sich bei diesen Klebemitteln um Klebstoffe. Diese Klebstoffe sind vorzugsweise hautverträglich und verursachen auch sonst keine Körperreaktionen, wie Kontaktallergien. Geeignete Klebstoffe für Verbandsmaterialien, wie Pflaster oder Tapeverbände, sind im Stand der Technik wohlbekannt. Das Verbandsmaterial kann mit dem Klebemittel getränkt sein oder, vorzugsweise einseitig, damit beschichtet sein. Vorzugsweise ist das Verbandsmaterial nicht vollständig mit dem Klebemittel versehen.

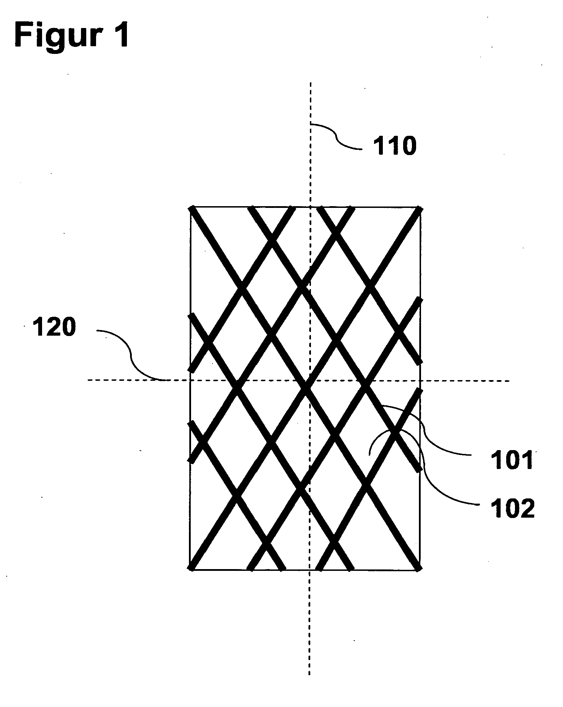

In einer bevorzugteren Ausführungsform des Verbandsmaterials ist das Klebemittel entlang der Begrenzungen von Rautenflächen (102), vorzugsweise in Form von Spreißen (101) angebracht, so dass die Rautenflächen der von den Begrenzungen gebildeten Rauten frei von Klebemittel verbleiben. Ein entsprechendes Verbandsmaterial ist in Figur 1 gezeigt. Am meisten bevorzugt sind die Rautenflächen dann mit den Wirkstoffen oder Wirkstoffgemischen versehen. Das Verbandsmaterial kann hierbei mit Lösungen der entsprechenden Wirkstoffe getränkt sein oder damit beschichtet sein, wobei eine Beschichtung bevorzugt ist.

In einer weiteren bevorzugten Ausführungsform des Verbandsmaterials ist das Klebemittel flächig auf das Trägermaterial aufgebracht. In dieser Ausführungsform werden die Wirkstoffe vorzugsweise direkt in ein geeignetes Klebemittel eingebracht bevor dieses auf das Trägermaterial aufgebracht wird. Geeignete Klebemittel, die die pharmazeutischen Eigenschaften der Wirkstoffe nicht beeinträchtigen, sind im Stand der Technik bekannt. Alternativ können die Wirkstoffe oder Wirkstoffmischungen nachträglich z.B. als Film auf ein bereits auf das Trägermaterial aufgebrachtes Klebemittel aufgebracht werden.

In einer weiteren bevorzugten Ausführungsform des erfindungsgemäßen Verbandsmaterials ist dieses entlang einer ersten Achse (110) bis zu 95 % dehnbar. Der Verlauf der ersten Achse des Verbandsmaterials ist in Figur 1 dargestellt. Vorzugsweise ist die erste Achse im Sinne der Erfindung die Längsachse des Verbandsmaterials, die sich dadurch auszeichnet, dass bei aufgebrachtem Verbandsmaterial parallel zu einer gedachten Achse entlang der Hautoberfläche und, falls ein Verbandmaterial mit rechteckiger Form, z.B. ein Band, als Verbandsmaterial verwendet wird, parallel zur längeren der Seitenflächen des Rechtecks verläuft.

In einer weiteren bevorzugten Ausführungsform des erfindungsgemäßen Verbandsmaterials ist das Verbandsmaterial entlang einer zweiten Achse (120) um höchstens 5 % dehnbar. Vorzugsweise ist die zweite Achse im Sinne der Erfindung die Querachse des Verbandsmaterials, die sich dadurch auszeichnet, dass bei aufgebrachtem Verbandsmaterial parallel zu einer gedachten Achse entlang der Hautoberfläche und, falls ein Verbandmaterial mit rechteckiger Form, z.B. ein Band, als Verbandsmaterial verwendet wird, parallel zur kürzeren der Seitenflächen des Rechtecks verläuft.

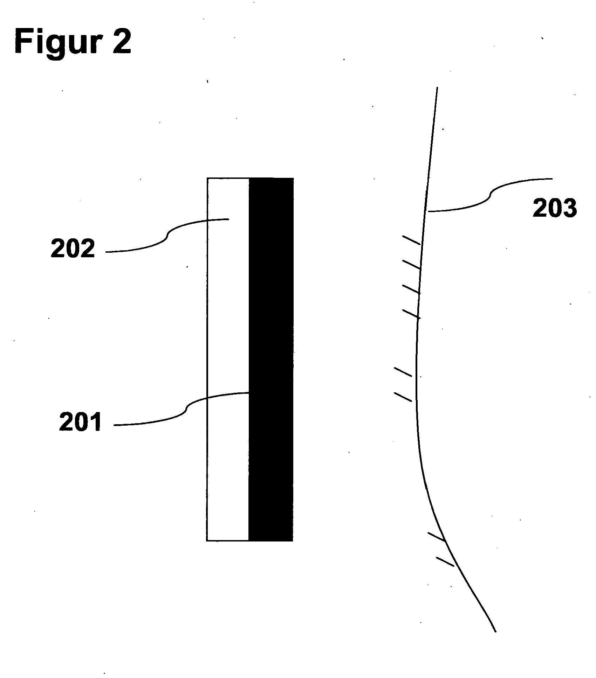

Bevorzugt im Rahmen der vorliegenden Erfindung ist auch ein Verbandsmaterial, bei dem das Trägermaterial ein erstes Trägermaterial (201) aufweist, das mit der Haut in Kontakt gebracht werden soll und ein zweites Trägermaterial (202) für die Außenseite des Verbands. Bevorzugt umfasst das erste Trägermaterial (201) Zinkoxid, Kautschuk und Acryl, wobei Zinkoxid vorzugsweise in 1 bis 10 %, besonders bevorzugt in 5 % (w/w), Kautschuk vorzugsweise in 1 bis 10%, besonders bevorzugt 5 % (w/w) und Acryl bevorzugt in 80 bis 98 %, besonders bevorzugt 90 % (w/w) vorliegt. Die Anteile an Zinkoxid, Kautschuk und Acryl in dem ersten Trägermaterial sollen sich im Rahmen dieser bevorzugten Ausführungsform jedoch immer auf 100 % (w/w) aufaddieren. Das zweite Trägermaterial (202) ist vorzugsweise Baumwolle, besonders bevorzugt reine Baumwolle (100 %). Ein bevorzugtes Trägermaterial im Sinne der Erfindung ist in Figur 2 gezeigt und im Ausführungsbeispiel beschrieben.

Die Erfindung betrifft auch ein Arzneimittel umfassend ein erfindungsgemäßes Verbandsmaterial wie zuvor beschrieben. Vorzugsweise wird das Verbandsmaterial bei der Arzneimittelformulierung steril verpackt. Wenn als Verbandsmaterial ein Band (z.B. Tape) eingesetzt werden soll, wird das Verbandsmaterial vorzugsweise aufgerollt auf einer Rolle als Arzneimittel bereitgestellt. Die Dosierung der von dem Verbandsmaterial umfassten Wirkstoffe wurde bereits zuvor beschrieben. Besonders bevorzugt wird das Arzneimittel so formuliert, dass es als Tapeverband eingesetzt werden kann. Für ein solches Arzneimittel wird ein rechteckiges Band aus Verbandsmaterial, vorzugsweise von 5 bis 15 m Länge, am meisten bevorzugt 10 m Länge aufgerollt bereitgestellt.

Die Erfindung betrifft schließlich die Verwendung eines erfindungsgemäßen Verbandsmaterials wie zuvor beschrieben zur Herstellung eines Arzneimittels zur Behandlung von Erkrankungen oder Störungen des Bewegungsapparates, vorzugsweise von muskulärem Schmerzsyndrom, Muskelverkrampfungen, Gelenkschmerzen und Gelenkverstauchungen, Sehnen- und Bänderbeschwerden, rheumatischen Beschwerden, Prellungen, Überdehnungen, Muskelfaserrissen, Bandrupturen, Zerrungen, frischen Verletzungen mit ausgeprägten Hämatomen, Fingerstauchungen, Skidaumen, Tennis- und Golfellenbogen, Impingmentsyndrom, Insertionsendopathie, Adduktorenverletzungen, Kniegelenksergüssen, Kniegelenks-beschwerden, Achillessehnenbeschwerden, Fersensporn, Sprunggelenksbeschwerden, Vor- und Mittelfußschmerzen (Längs- und Quergewölbe), Handgelenksbeschwerden, Halswirbelsäulen, Brustwirbelsäulen oder Lendenwirbelsäulenbeschwerden, Migräne oder Lymphödeme. Das Verbandsmaterial wird hierbei vorzugsweise als Tapeverband eingesetzt.

Die Figuren zeigen:
- Figur 1:: Sicht auf die Hautkontaktfläche eines erfindungsgemäßen Trägermaterials, wobei Begrenzungen der Rautenflächen (101), die mit Klebemittel versehen sein sollen, die klebemittelfreien Rautenflächen (102) sowie die erste (Längs-)achse (110) und die zweite (Quer-)achse (120) gezeigt werden.
- Figur 2:: Die Figur zeigt einen Querschnitt durch ein erfindungsgemäßes Trägermaterial, das aus einem ersten Trägermaterial (201) und einem zweiten Trägermaterial (202) besteht. Das erste Trägermaterial (201) soll hierbei mit der Hautoberfläche (203) in Kontakt gebracht werden.

Die Erfindung wird nun anhand der Ausführungsbeispiele illustriert. Die Ausführungsbeispiele sollen hierbei den Schutzumfang nicht begrenzen.

### Beispiel 1: Tapeverbandsmaterial

Ein etwa 10 m langes Band von 4,5 cm Breite besteht aus einem Oberflächenmaterial und einem hautseitigen aufgebrachten Material. Das Oberflächenmaterial ist 100 % Baumwolle. Das hautseitige Material besteht zu 5 % aus Zinkoxid, 5 % aus Kautschuk und 90 % aus Acryl. Ein Kleber ist auf dem hautseitigen Material in Streifen angebracht, wobei die Streifen so angebracht sind, dass sie rautenförmige Flächen begrenzen. Innerhalb dieser Rautenflächen werden verdünnte Extrakte von Arnica montana, Rhus toxicodendron und Ruta graveolens aufgebracht. Die Extrakte sind Arnica D 12, Rhus toxicodendron D 12 und Ruta graveolens D12. Das Verbandsmaterial ist so verarbeitet, dass es luftdurchlässig und wasserabweisend ist. Das Verbandsmaterial erlaubt 95 % Längsdehnung und 5 % Querdehnung. Die für das Verbandsmaterial verwendeten Substanzen sind hypoallergen. Das Verbandsmaterial ist in den Farben rot, blau, gelb gehalten.

Das Verbandsmaterial wird als Tapeverband bei Verletzungen und Störungen des Bewegungsapparates eingesetzt. Es wurden bereits verschiedentlich sehr gute Heilerfolge erzielt. Insbesondere konnte die Lymphdrainage und eine bessere Blutversorgung der betroffenen Teile des Bewegungsapparates erzielt werden sowie Muskelatrophien vermieden werden.

### Beispiel 2: Fallstudie

Patient: männlich, 30 Jahre, Sportler
Anamnese: Zog sich beim Tennis spielen eine Achillessehnenteilruptur zu
Diagnostik: mit Hilfe einer Ultraschalluntersuchung wurde Teilruptur festgestellt
vorherige Therapie: Adimedschuh mit Fersenkeilen für 4-6 Wochen (innerhalb dieser Zeit Keilveränderung bis zur neutral-null Stellung des Sprunggelenkes)
Patient klagt noch Wochen nach der Heilungsphase, dass der Fuß dicker war, über morgendlichen Anlaufschmerz und Instabilitätsgefühl.

### Therapie mit Taperverbandsmaterial aus Beispiel 1:

Durch Anlegen des Verbandes wurden der morgendliche Anlaufschmerz und das Ödem innerhalb 2 Tagen behoben. Nach 1 Woche wurde der Verband gewechselt und nach der 2. Woche komplett entfernt. Der Patient konnte seinen sportlichen Aktivitäten wieder normal nachgehen.

Durch die Wirkung der Wirkstoffe auf dem Tapeverband wurden Blut und Blutgefäße und lymphathisches System positiv beeinflusst. Durch die Erhöhung der Durchblutung wird die Regeneration des Gewebes verbessert (die bei einer Verletzung des Muskel-Kapsel-Bandapparates durch das Trauma ja nicht optimal versorgt ist). Durch das Auftragen des Tapeverbands, welcher sich den Hautbewegungen durch seine Flexibilität anpasst, wurde mit jeder Bewegung des Patienten die Haut und Schmerzrezeptoren und das zirkulatorische System aktiviert, so dass die Wirkstoffe lokal einwirken konnten. Der Abtransport von Gewebeflüssigkeit wurde erhöht. Die Zirkulation wurde deutlich beschleunigt und somit der Heilungsprozess verkürzt. Durch eine schnellere Schmerzreduktion lässt sich eine schnellere Belastung ermöglichen, dadurch wurde wiederum eine Erhöhung des zirkulatorischen Systems bewirkt und gleichzeitig der Muskelaufbau unterstützt.

## Patentansprüche

1. Verbandsmaterial umfassend ein Trägermaterial sowie zumindest einen Wirkstoff aus jeweils Arnica montana, Rhus toxicodendron und Ruta graveolens.

2. Verbandsmaterial nach Anspruch 1, wobei der zumindest ein Wirkstoff aus Arnica montana, ein verdünnter Extrakt ist, der aus einem Teil Extrakt auf 10¹² Teilen eines pharmazeutisch verträglichen Lösungsmittels besteht (Arnica D 12).

3. Verbandsmaterial nach Anspruch 1 und 2, wobei der zumindest eine Wirkstoff aus Rhus toxicodendron, ein verdünnter Extrakt, der aus einem Teil Extrakt auf 10¹² Teilen eines pharmazeutisch verträglichen Lösungsmittels besteht (Rhus toxicodendron D12).

4. Verbandsmaterial nach einem der Ansprüche 1 bis 3, wobei der zumindest eine Wirkstoff aus Ruta graveolens, ein verdünnter Extrakt ist, der aus einem Teil Extrakt auf 10¹² Teilen eines pharmazeutisch verträglichen Lösungsmittels besteht (Ruta graveolens D 12).

5. Verbandsmaterial umfassend ein Trägermaterial sowie zumindest einen Wirkstoff aus jeweils Flos cathaimi, Ruta graveolens und Rhus delavayi.

6. Verbandsmaterial umfassend ein Trägermaterial, vorzugsweise nach einem der Ansprüche 1 bis 5, und zumindest einen weiteren Wirkstoff ausgewählt aus der Gruppe bestehend aus: Bryonia alba, Bryonia dioica, Mixturen aus Kirschpflaumenblüten, Blüten der Weißen Waldrebe, Blüten des Drüsen tragenden Springkraut, Blüten des Gelben Sonnenröschens und Blüten des Goldigen Milchsterns, Hypericum perforatum, Apis mellifica, Calendula, Strontium carbonicum, Agaricus muscarius, Anacardium orientale, Lendium palustris, Bellis percunis, Acidum sulforicum, Hamamelis virginica und Diclofenac.

7. Verbandsmaterial nach einem der Ansprüche 1 bis 5, das ein Klebemittel zum Befestigen des Verbandmaterials an der Haut aufweist.

8. Verbandsmaterial nach Anspruch 7, wobei das Klebemittel entlang der Begrenzungen von Rautenflächen (102) angebracht ist und die Rautenflächen frei von Klebemittel verbleiben.

9. Verbandsmaterial nach Anspruch 8, wobei die Rautenflächen mit dem zumindest einen Wirkstoff aus jeweils Arnica montana, Rhus toxicodendron, Ruta graveolens versehen sind.

10. Verbandsmaterial nach einem der Ansprüche 1 bis 9, wobei das Verbandsmaterial entlang einer ersten Achse (110) um bis zu 95% dehnbar ist.

11. Verbandsmaterial nach einem der Ansprüche 1 bis 10, wobei das Verbandsmaterial entlang einer zweiten Achse (120) um höchsten 5% dehnbar ist.

12. Verbandsmaterial nach einem der Ansprüche 1 bis 11, wobei das Trägermaterial ein erstes Trägermaterial (201) aufweist, das mit der Haut in Kontakt gebracht werden soll und ein zweites Trägermaterial (202) für die Außenseite des Verbands aufweist.

13. Verbandsmaterial nach Anspruch 12, wobei das erste Trägermaterial (201) Zinkoxid, Kautschuk und Acryl umfasst.

14. Verbandsmaterial nach Anspruch 13, wobei Zinkoxid in 1 bis 10% (W/W), Kautschuk in 1 bis 10% (W/W) und Acryl in 80 bis 98% (W/W) vorliegt.

15. Verbandsmaterial nach Anspruch 13 oder 14, wobei Zinkoxid in 5% (W/W), Kautschuk in 5% (W/W) und Acryl in 90% (W/W) vorliegt.

16. Verbandsmaterial nach einem der Ansprüche 12 bis 15, wobei das zweite Trägermaterial (202) Baumwolle umfasst.

17. Arzneimittel umfassend ein Verbandsmaterial nach einem der Ansprüche 1 bis 16.

18. Verwendung eines Verbandsmaterials nach einem der Ansprüche 1 bis 16 zur Herstellung eines Arzneimittels zur Behandlung von muskulärem Schmerzsyndrom, Muskelverkrampfungen, Gelenkschmerzen und Gelenkverstauchungen, Sehnen- und Bänderbeschwerden, rheumatischen Beschwerde, Prellungen, Überdehnungen, Muskelfaserrisse, Bandrupturen, Zerrungen, frische Verletzungen mit ausgeprägtem Hämatom, Fingerstauchungen, Skidaumen, Tennis- und Golfellenbogen, Impingmentsyndrom, Insertionsendopathie, Adduktorenverletzungen, Kniegelenksergüsse, Kniegelenkbeschwerden, Achillessehnenbeschwerden, Fersenspom, Sprunggelenksbeschwerden, Vor- und Mittelfußschmerzen (Längs- und Quergewölbe), Handgelenksbeschwerden, HWS, BWS, LWS Beschwerden, Migräne, Lymphödeme.

19. Arzneimittel nach Anspruch 17 oder Verwendung nach Anspruch 18, wobei das Verbandsmaterial als Tape-Verband eingesetzt wird.

## Claims

1. A dressing material comprising a support material and at least one active substance from in each case Arnica montana, Rhus toxicodendron and Ruta graveolens.

2. The dressing material as claimed in claim 1, where the at least one active substance from Arnica montana is a dilute extract which consists of a partial extract in 10¹² parts of a pharmaceutically acceptable solvent (Arnica D 12).

3. The dressing material as claimed in claim 1 and 2, where the at least one active substance from Rhus toxicodendron, a dilute extract, which consists of a partial extract in 10¹² parts of a pharmaceutically acceptable solvent (Rhus toxicodendron D12).

4. The dressing material as claimed in any of claims 1 to 3, where the at least one active substance from Ruta graveolens is a dilute extract which consists of a partial extract in 10¹² parts of a pharmaceutically acceptable solvent (Ruta graveolens D12).

5. A dressing material comprising a support material and at least one active substance from in each case Flos cathaimi, Ruta graveolens and Rhus delavayi.

6. A dressing material comprising a support material, preferably as claimed in any of claims 1 to 5, and at least one further active substance selected from the group consisting of: Bryonia alba, Bryonia dioica, mixtures of cherry plum flowers, flowers of white clematis, flowers of impatiens glandulifera, flowers of the yellow sunrose and flowers of the Star of Bethlehem, Hypericum perforatum, Apis mellifica, Calendula, Strontium carbonicum, Agaricus muscarius, Anacardium orientale, Lendium palustris, Bellis percunis, Acidum sulforicum, Hamamelis virginica and diclofenac.

7. The dressing material as claimed in any of claims 1 to 5, which includes an adhesive for attaching the dressing material to the skin.

8. The dressing material as claimed in claim 7, where the adhesive is applied along the margins of rhombic areas (102), and the rhombic areas remain free of adhesive.

9. The dressing material as claimed in claim 8, where the rhombic areas are provided with the at least one active substance from in each case Arnica montana, Rhus toxicodendron, Ruta graveolens.

10. The dressing material as claimed in any of claims 1 to 9, where the dressing material is up to 95% extensible along a first axis (110).

11. The dressing material as claimed in any of claims 1 to 10, where the dressing material is no more than 5% extensible along a second axis (120).

12. The dressing material as claimed in any of claims 1 to 11, where the support material includes a first support material (201) which is to be brought into contact with the skin, and includes a second support material (202) for the outside of the dressing.

13. The dressing material as claimed in claim 12, where the first support material (201) comprises zinc oxide, rubber and acrylic.

14. The dressing material as claimed in claim 13, where 1 to 10% (w/w) zinc oxide, 1 to 10% (w/w) rubber and 80 to 98% (w/w) acrylic are present.

15. The dressing material as claimed in claim 13 or 14, where 5% (w/w) zinc oxide, 5% (w/w) rubber and 90% (w/w) acrylic are present.

16. The dressing material as claimed in any of claims 12 to 15, where the second support material (202) comprises cotton.

17. A medicament comprising a dressing material as claimed in any of claims 1 to 16.

18. The use of a dressing material as claimed in any of claims 1 to 16 for producing a medicament for the treatment of muscular pain syndrome, muscle cramps, joint pain and joint sprains, tendon and ligament complaints, rheumatic complaints, contusions, overstretchings, muscle fiber tears, ruptured ligaments, strains, fresh injuries with pronounced hematoma, finger sprains, skier's thumb, tennis and golf elbows, impingment syndrome, insertion endopathy, adductor injuries, knee joint effusions, knee joint complaints, Achilles tendon complaints, heel spur, ankle complaints, forefoot and metatarsal pain (longitudinal and transverse arch), wrist complaints, cervical spine, thoracic spine or longitudinal spine complaints, migraine or lymphedemas.

19. The medicament as claimed in claim 17 or use as claimed in claim 18, where the dressing material is employed as tape dressing.

## Revendications

1. Matériau de bandage comprenant un matériau de support ainsi qu'au moins un principe actif composé respectivement d'Arnica Montana, de Rhus toxicodendron et de Ruta graveolens.

2. Matériau de bandage selon la revendication 1, le au moins un principe actif composé d'Arnica Montana étant un extrait dilué qui se compose d'une partie d'extrait sur 10¹² parties d'un solvant compatible au niveau pharmaceutique (Arnica D12).

3. Matériau de bandage selon la revendication 1 et 2, le au moins un principe actif composé de Rhus toxicodendron étant un extrait dilué qui se compose d'une partie d'extrait sur 10¹² parties d'un solvant compatible au niveau pharmaceutique (Rhus toxicodendron D12).

4. Matériau de bandage selon l'une des revendications 1 à 3, le au moins un principe actif composé de Ruta graveolens étant un extrait dilué qui se compose d'une partie d'extrait sur 10¹² parties d'un solvant compatible au niveau pharmaceutique (Ruta graveolens D12).

5. Matériau de bandage comprenant un matériau de support ainsi qu'au moins un principe actif composé respectivement de Flos cathaimi, de Ruta graveolens et de Rhus delavayi.

6. Matériau de bandage comprenant un matériau de support, de préférence selon l'une des revendications 1 à 5, et au moins un autre principe actif choisi dans le groupe se composant de : bryone blanche, bryone dioïque, mélanges des fleurs de prunier myrobalan, fleurs de clématites blanches, fleurs de glandes de balsamine de l'Himalaya, fleurs d'hélianthème jaunes et fleurs d'ornithogale dorées, Hypericum perforatum, Apis mellifica, Calendula, strontium carbonicum, Agaricus muscarius, Anacardium orientale, Lendium palustris, Bellis percunis, Acidum sulforicum, Hamamelis virginica et Diclofénac.

7. Matériau de bandage selon l'une des revendications 1 à 5 qui comprend un adhésif servant à fixer le matériau de bandage sur la peau.

8. Matériau de bandage selon la revendication 7, l'adhésif étant appliqué le long des bords des surfaces en losange (102) et les surfaces en losange restant dépourvues d'adhésif.

9. Matériau de bandage selon la revendication 8, les surfaces en losange étant prévues avec le au moins un principe actif composé respectivement d'Arnica Montana, de Rhus toxicodendron, de Ruta graveolens.

10. Matériau de bandage selon l'une des revendications 1 à 9, le matériau de bandage étant expansible de jusqu'à 95 % le long d'un premier axe (110).

11. Matériau de bandage selon l'une des revendications 1 à 10, le matériau de bandage étant expansible de maximum 5 % le long d'un second axe (120).

12. Matériau de bandage selon l'une des revendications 1 à 11, le matériau de support comprenant un premier matériau de support (201) qui doit être mis au contact de la peau et un second matériau de support (202) pour le côté extérieur du bandage.

13. Matériau de bandage selon la revendication 12, le premier matériau de support (201) comprenant de l'oxyde de zinc, du caoutchouc et de l'acrylique.

14. Matériau de bandage selon la revendication 13, l'oxyde de zinc se trouvant dans une zone de 1 à 10 % (M/M), le caoutchouc dans une zone de 1 à 10 % (M/M) et l'acrylique dans une zone de 80 à 98 % (M/M).

15. Matériau de bandage selon la revendication 13 ou 14, l'oxyde de zinc représentant 5 % (M/M), le caoutchouc 5 % (M/M) et l'acrylique 90 % (M/M).

16. Matériau de bandage selon l'une quelconque des revendications 12 à 15, le second matériau de support (202) comprenant du coton.

17. Médicament comprenant un matériau de bandage selon l'une des revendications 1 à 16.

18. Utilisation d'un matériau de bandage selon l'une des revendications 1 à 16 destiné à fabriquer un médicament servant à traiter un syndrome de douleurs musculaires, des contractions musculaires, des douleurs et des foulures articulaires, des tendons et ligaments douloureux, des douleurs rhumatismales, des contusions, des distensions, des déchirures de fibres musculaires, des ruptures de ligament, des claquages, des blessures récentes avec un hématome prononcé, des doigts écrasés, des pouces de skieur, des coudes de joueur de tennis et de golf, un syndrome de coincement, une intro-affection, des blessures aux adducteurs, des épanchements de l'articulation du genou, des douleurs de l'articulation du genou, des douleurs du tendon d'Achille, une exostose sous-calcanéenne, des douleurs de l'articulation du pied, des douleurs du tarse et du métatarse (voûte plantaire longitudinale et transversale), des douleurs du poignet, des douleurs de la colonne vertébrale cervicale, de la colonne thoracique, de la portion abdominale de la colonne vertébrale, des migraines, un oedème lymphatique.

19. Médicament selon la revendication 17 ou utilisation selon la revendication 18, le matériau de bandage étant utilisé comme bande adhésive.
